(19) Europäisches Patentamt
European Patent Office
Office européen des brevets

(11) Veröffentlichungsnummer: **0 261 336 B1**

(12) # EUROPÄISCHE PATENTSCHRIFT

(45) Veröffentlichungstag der Patentschrift: **02.01.92**

(51) Int. Cl.⁵: **C07D 339/04**, C07D 303/14, C07C 309/63, C07C 33/035

(21) Anmeldenummer: **87110526.8**

(22) Anmeldetag: **21.07.87**

(54) **Verfahren zur Herstellung enantiomerenreiner R-( + )-alpha-Liponsäure und S-(-)-alpha-Liponsäure (Thioctsäure) sowie Nonen- beziehungsweise Mesylderivate als Zwischenprodukte hierfür.**

(30) Priorität: **27.08.86 DE 3629116**

(43) Veröffentlichungstag der Anmeldung:
**30.03.88 Patentblatt 88/13**

(45) Bekanntmachung des Hinweises auf die Patenterteilung:
**02.01.92 Patentblatt 92/01**

(84) Benannte Vertragsstaaten:
**BE CH DE FR GB IT LI NL**

(56) Entgegenhaltungen:
**GB-A- 938 627**

**JOURNAL OF THE CHEMICAL SOCIETY, CHEM. COMMUN., 1986, Seiten 1408-1409, London, GB; P.C. BULMAN PAGE et al.: "Enantioselective synthesis of R-( + )-alpha-lipoic acid"**

(73) Patentinhaber: **ASTA Pharma Aktiengesellschaft**
**Weismüllerstrasse 45**
**W-6000 Frankturt am Main 1(DE)**

(72) Erfinder: **Sutherland, Ian O.**
**The Chase Melloncroft Drive West**
**Caldy Wirral Merseyside L48 2JD(GB)**
Erfinder: **Page, Philip C.B.**
**47 Beryl Road**
**Noctorum Birkenhead L43 9RS(GB)**
Erfinder: **Rayner, Christopher M.**
**26 September Road Tuesbrook**
**Liverpool L6(GB)**

**EP 0 261 336 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von enantiomerenreiner 1,2-Dithiolan-3-pentansäure (Thioctsäure, α-Liponsäure) sowie Nonen-beziehungsweise Mesylderivate als Zwischenprodukte hierfür. D,L-Thioctsäure wird in Form des racemischen Gemisches als pharmazeutisches Präparat zur Behandlung von akuten und chronischen Lebererkrankungen sowie Vergiftungen eingesetzt. Die optisch aktive R-(+)-α-Liponsäure ist ein Naturstoff, der in geringer Konzentration im tierischen und menschlichen Organismus vorkommt. R-(+)-α-Liponsäure wirkt als Coenzym bei der oxidativen Decarboxylierung von α-Ketosäuren. Die R-Konfiguration der natürlich verkommenden α(+)-Liponsäure wurde durch eine enantioselektive Synthese des (-)-Antipoden ausgehend von S-Malonsäure bestätigt (M.H. Brookes, B.T. Golding, D.A. Howes, A.T. Hudson J.Chem. Soc., Chem. Commun. 1983, 1051).
Es liegen Hinweise vor, daß die beiden enantiomeren Formen der α-Liponsäure nicht die gleiche biologische Aktivität besitzen, sondern daß das S(-)-Enantiomere eine geringere Aktivität aufweist (J.C.Gunsalus, L.S. Barton, W.Gruber J.Am.Chem.Soc. 78, 1763 (1956). Für eine rationelle Pharmakotherapie ist es deshalb notwendig, die stärker wirksame enantiomere Form der α-Liponsäure einzusetzen. Die bekannten Verfahren zur Herstellung von α-Liponsäure ergeben lediglich ein racemisches Gemisch (siehe DE-OS 35 12 911.5 und dort zitierte Literatur). Ein Verfahren zur Racematspaltung mit D(-)Arabinose ist zwar bekannt (L.G.Chebotareva, A.M. Yurkerische Khim.-Farm.

Zh. 14(9). 92-99 1980, C.A. 94 (13), 103 722 g), jedoch sind die erzielten Ausbeuten nur gering und das Verfahren ist unwirtschaftlich.
Die erste bekannte asymmetrische Synthese zur Herstellung von R(+)-α-Liponsäure geht von einer 7-stufigen Reaktionsfolge aus (J.D. Elliott, J. Steele, W.S. Johnson Tetrahedron Lett., 1985 26, 2535). Hierbei muß als Ausgangssubstanz teures chirales 2,4-Pentandiol eingesetzt werden, so daß eine wirtschaftliche Synthese von enantiomerenreinerα-Liponsäure nicht möglich ist. Zugleich gelingt die Herstellung nur einer enantiomeren Form.
Der Erfindung liegt die Aufgabe zugrunde, eine neue wirtschaftliche Synthese zur Herstellung von enantiomerenreiner R(+)-α-Liponsäure und S(-)-α-Liponsäure bereitzustellen.

Die Synthese geht von einer achiralen Ausgangssubstanz aus, so daß in einem späteren Reaktionsschritt durch Reaktion mit einer chiralen Substanz wahlweise entweder R(+)-α-Liponsäure oder S(-)-α-Liponsäure hergestellt werden kann. Die Synthese erfolgt in einer 6-stufigen Reaktionsfolge mit einer Gesamtausbeute von 22 % unter Kontrolle der absoluten Konfiguration im Asymmetriezentrum am C-6 Atom der α-Liponsäure.

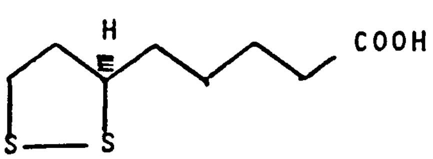

R-(+)-α-Liponsäure

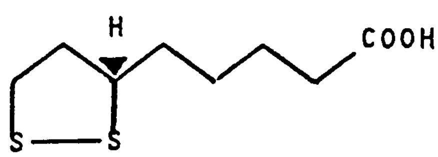

S-(-)-α-Liponsäure

Die enantioselektive Synthese verwendet als Ausgangsmaterial leicht zugänglichen Propargylalkohol, der mit Lithium in flüssigem Ammoniak und 1-Brom-5-hexen in einer Eintopfreaktion und nachfolgender Reduktion mit Lithium des in situ gebildeten disubstituierten Acetylens E-Nona-2,8-dien-1-ol(II) ergibt (J.W. Patterson Synthesis 337, 1985).

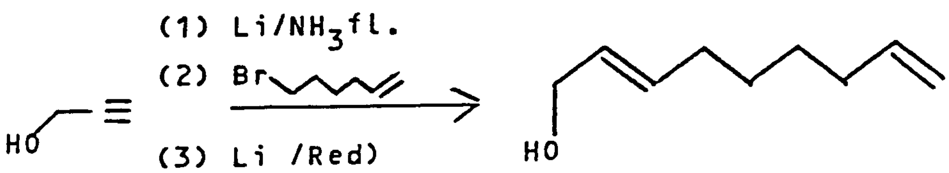


II

Durch Gaskapillarchromatographie wird bestätigt, daß 100% als E-Isomeres vorliegt.
Die katalytische enantioselektive Epoxydation des E-Nona-2,8-dien-1-ols(II) erfolgt mit Tetraisopropylorthotitanat und chiralen Tartraten, insbesondere mit Di-isopropyltartrat, und tert.-Butylhydroperoxid in Halogenkohlenwasserstoffen, insbesondere in Methylenchlorid oder Chloroform bei Temperaturen zwischen -25° C bis -5° C, nach der Methode von K.B. Sharpless et. al. (EP-OS 0 046 033, K.B. Sharpless et al.
Pure Appl. Chem. 1983, 55, 1823 und dort zitierte Literatur). Unter Verwendung von L(+)-Diisopropyltartrat als chirales Reagens erhält man 2S, 3S-2-Epoxy-1-hydroxynon-8-en(III),während man mit D(-)-Diisopropyltartrat das entsprechende enantiomere 2R, 3R-2-Epoxy-1-hydroxynon-8-en erhält.

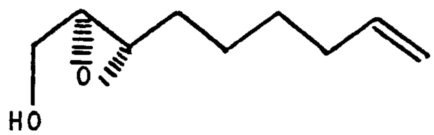

(III)

2S, 3S

Man geht bevorzugt von 0,1 Äquivalenten Tetraisopropylorthotitanat, 0,12 Äquivalenten L(+)-Diisopropyltartrat, 1,2 Äquivalenten tert.-Butylhydroperoxid unter Verwendung von Methylenchlorid als Lösungsmittel aus.
Die katalytische Menge chirales Reagens kann bei Verwendung von 4 Å Molekularsieben noch weiter reduziert werden (R.M. Hansons, K.B. Sharpless J.Org.Chem. 1922, 51. 1986).

Die optische Reinheit des 2S, 3S-2-Epoxy-1-hydroxynon-8-ens(III) mit 96% ee. kann durch Gaskapillarchromatographie und durch $^{19}$F-NMR Analyse des Esters aufgrund der Reaktion mit S-(-)-α-methoxy-α-trifluormethylphenylacetylchlorid (J.A.Dale, D.L. Dull, H.S. Mosher J. Org.Chem. 1969, 34, 2543) bestimmt werden. Die Reduktion des Epoxyalkohols (III) mit einem metallorganischen Reduktionsmittel wie beispielsweise mit 1,5 bis 3-fachem molaren Überschuß Natrium-bis(2-methoxy-ethoxy)-aluminiumhydrid(Red-Al® ) in einem inerten organischen Lösungsmittel, wie beispielsweise Tetrahydrofuran oder Ether bei Temperaturen von zwischen -20° C und +5° C ergibt in selektiver Bildung unter Erhalt der Konfiguration am C-3 Atom 3S-1,3-Dihydroxynon-8-en(IV)

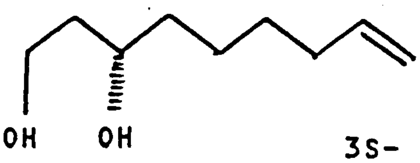

3S- IV

Die Reduktion erfolgt nach der Methode von J.M.Finan, Y.Kishi Tetrahedron Lett. 1982, 23, 2719.

Das enantiomerenreine Diol(IV) wird mit Mesyl- oder Tosylchlorid in einem inerten organischen Lösungsmittel, wie zum Beispiel Methylenchlorid oder Chloroform bei Temperaturen zwischen -20° C und +5° C unter Erhalt der Konfiguration am C-3 Atom in das 3S-Dimesyl/tosylat(V) übergeführt, um die -OH-Gruppen bei der nachfolgenden Oxidation zu schützen und in einer späteren Reaktionsstufe die Disulfid-Gruppen in einfacher Weise einführen zu können.

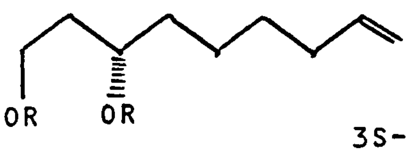

3S- V

$R = SO_2CH_3$, $SO_2-C_6H_4-CH_3$

Die Dimesylat/tosylatbildung erfolgt nach der allgemeinen Methode von R.K. Crossland, K.L.Servis J.Org. Chem. 1970, 35, 3195.
Die weitere Reaktionsfolge ergibt unter Oxidation der terminalen Doppelbindung nach der Methode von P.H.J. Carlsen, T.Katsuki, V.S.Martin, K.B.Sharpless J.Org.Chem. 1981, 46, 3936 mit katalytischen Mengen

(ca. 1 Mol%) in situ gebildetem Rutheniumtetroxid aus Rutheniumtrichloridhydrat und Natriumperjodat unter Erhalt des Asymmetriezentrums 6S-6,8-Dimesyl/tosyloxyoctansäure(VI).

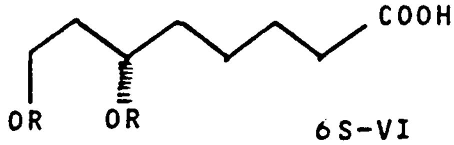

6S-VI

$R=SO_2CH_3$
$SO_2-C_6H_4-CH_3$

Eine katalytische Menge Rutheniumtrichloridhydrat ist hierbei ausreichend, während Natriumperjodat in einer 4-6-fachen Menge bezogen auf das Alken(IV)vorliegen muß. Das Reaktionsmedium ist bevorzugt ein Lösungsmittelgemisch wie beispielsweise Kohlenstofftetrachlorid, Acetonitril oder Chloroform.

Der Austausch der beiden Mesyl beziehungsweise Tosylgruppen unter Einführung der Disulfidbindung gelingt mit Natriumsulfid-nonahydrat, Schwefel, Kaliumhydroxid in einem organischen Lösungsmittel wie zum Beispiel Ethanol oder Dimethylformamid unter Inversion der Konfiguration am C-6 Atom unter Bildung der enantiomerenreinen R(+)-α-Liponsäure(VII) (siehe M.H. Brookes, B.T. Golding, D.A.Howes, A.T. Hudson J.Chem.Soc., Chem. Commun. 1983 1051 und E.L.Eliel et al. Tetrahedron Lett. 1980, 331) R(+)-α-Liponsäure(VII) zeigt einen Schmelzpunkt von 44 - 46 °C $[\alpha]_D^{28}$ = + 107° (c = 0,82 in Benzol) und zeigt Übereinstimmung mit den Literaturdaten (Schmelzpunkt 43 - 45 °C $[\alpha]_D^{23}$ = + 108° (c = 0,91 in Benzol) siehe J.D.Elliott, J.Steele, W.S. Johnson Tetrahedron Lett. 1985, 26 2535)

Unter Verwendung des chiralen Hilfsreagens D-(-)-Diisopropyltartrat bei der Epoxydierung zu dem Epoxyalkohol(III) erhält man in gleicher Ausbeute die enantiomerenreine S(-)-α-Liponsäure.

(E)-Nona-2,8-dien-1-ol (II)

100 ml flüssiger Ammoniak und eine katalytische Menge Eisen(III)nitrat (5 mg) werden in einen 250 ml 3-Halskolben vorgelegt, der mit einem Rährer, Trockeneiskondensator, Tropftrichter mit Druckausgleich und mit einer Argon/Ammoniak-Spülung versehen ist. Zu dieser Mischung gibt man portionsweise 1,88 g (0,268 M) Lithium in der Weise, daß die blaue Farbe zwischen den Zugaben verschwindet. Zu der Reaktionsmischung gibt man 7,21 g (7,49 ml, 0,129 M) Propargylalkohol in 20 ml trockenem Tetrahydrofuran während 25 Minuten und läßt nach der Zugabe während 1 1/2 Stunden unter Rückflußkochen] nachreagieren. Anschließend gibt man 14,0 g (10,86 ml, 85,8 mM) 1-Brom-5-hexen in 30 ml trockenem Tetrahydrofuran während 30 Minuten hinzu und läßt nochmals unter Rückflußkochen während 2 1/2 Stunden nachreagieren. Dann gibt man 2,05 g (0,298 M) Lithium portionsweise zu der Reaktionsmischung und

läßt noch 1 Stunde nachreagieren. Anschließend erfolgt die Zugabe von Ammoniumchlorid in einer Menge, daß die blaue Farbe der Reaktionslösung verschwindet und die Hauptmenge des Ammoniaks verdampfen kann. Die Reaktionsmischung wird anschließend in 20 g Eis gegeben, und man läßt über Nacht die Mischung auf 25 °C erwärmen. Die Reaktionsmischung wird mit Äther extrahiert und über Magnesiumsulfat getrocknet. Nach dem Abziehen des Lösungsmittels erhält man durch Kugelrohrdestillation des Rückstands bei 105 °C/0,5 mm Druck 9,3 g (66,4 mM) E-Nona-2,8-dien-1-ol. Dies entspricht einer Ausbeute von 78%.

Durch gaschromatographische Analyse wird bestätigt, daß reines E-Isomeres vorliegt.

$C_9H_{16}O$: Analyse berechnet: C:77,09% H:11,50%

gefunden: C:77,30% H:11,49%

$^1$H-NMR($CDCl_3$): δ 5,90-5,50 (m,3H), δ 5,05-4,90 (m,2H) δ 4,07 (d,2H J = 4,0 Hz) δ 2,03 (s,4H) δ 1,85 (br., s, 1H verschwindet durch Schütteln mit $D_2O$) δ 1,40 (m,4H)

IR Spektrum (Film): 3350, 3090, 2915, 2860, 1675, 1640, 1460, 1440, 1230, 1090, 1000, 970, 910 $cm^{-1}$.

Die Banden bei 1000 $cm^{-1}$ und 970 $cm^{-1}$ zeigen eine E-substituierte Doppelbindung an.

2-Epoxy-1-hydroxynon-8-en(III)

In einem 250 ml Rundkolben werden 100 ml Methylenchlorid vorgelegt und auf -25 °C gekühlt. Das Reaktionsgefäß wird mit einem Magnetrührer ausgestattet und mit einem Schutzaufsatz versehen, wobei die Reaktionsapparatur mit Argonschutzgas gespült wird. 2,84 g (2,97 ml, 10 mM) Titantetraisopropylat werden

mittels einer Spritze in das Reaktionsgefäß zugegeben; nach weiteren 5 Minuten erfolgt die Zugabe von 2,81 g (2,52 ml, 12 mM) frisch destilliertem L(+)-Diisopropyltartrat.
Die Zugabe von 2,72 g (19,4 mM) (E)-Nona-2,8-dien-1-ol(II) in das Reaktionsgemisch erfolgt nach weiteren 5 Minuten, sodann erfolgt nach weiteren 5 Minuten die Zugabe von 6,9 ml einer 3,39 molaren Lösung in Toluol (23,4 mM) von tertiär-Butylhydroperoxid. Das Reaktionsgemisch läßt man bei -25°C 3 Tage nachreagieren. Anschließend gibt man das Reaktionsgefäß in ein Eisbad und fügt zu der Reaktionsmischung 10 ml (1 ml pro mM eingesetztes Titantetraisopropylat) gesättigte wässrige Natriumsulfatlösung und weitere 100 ml Diethyläther hinzu.
Die Reaktionslösung wird ca. 1 Stunde heftig gerührt bis sich die Lösung auf Zimmertemperatur erwärmt hat. Der gelatineartige,orangefarbene Niederschlag wird mittels einer Glasfritte abgetrennt und zweimal mit je 100 ml Ether gewaschen und durch Absaugen getrocknet. Aus den vereinigten Filtraten wird das Lösungsmittel verdampft, und der Rückstand wird in 100 ml Ether und 2 ml Dimethylsulfid aufgenommen, um überschüssiges tertiär-Butylhydroperoxid zu zerstören. Die Lösung wird eine Stunde gerührt, anschließend gibt man 50 ml 1 molares Natriumhydroxid hinzu und rührt weitere 3-4 Stunden.

Die Etherphase wird abgetrennt, mit 2 x 40 ml Wasser gewaschen, über Magnesiumsulfat getrocknet und nach Verdampfen des Lösungsmittels im Vakuum erhält man durch Kugelrohrdestillation bei 110°C/0,5 mm Druck 2,48 g (15,9 mM) 2S, 3S-2-Epoxy-1-hydroxynon-8-en(III). Dies entspricht einer Ausbeute von 82 %.
Die optische Reinheit beträgt 92% (Die Bestimmung erfolgt durch Gaskapillarchromatographie und durch $^{19}$F-NMR Analyse des Esters, der bei der Reaktion mit S-(-)-$\alpha$-methoxy-$\alpha$-trifluormethylphenylacetylchlorid gebildet wird - siehe J.A. Dale, D.L.Dull, H.S. Mosher J.Org.Chem. 1969, 34, 2543)
$C_9H_{16}O_2$: Analyse berechnet: C:69,19 % H:10,32%
gefunden: C:69,19 % H:10,12%
$^1$H-NMR ($CDCl_3$): $\delta$ 5,90 - 5,68 (m,1H), $\delta$ 5,07-4,90 (m,2H), $\delta$ 3,87 (AB,1H), $\delta$ 3,63 (AB,1H), $\delta$ 2,92 (d, J = 4Hz, 2H), $\delta$ 2,60 (br.,s,1H, verschwindet durch Austausch mit $D_2O$), $\delta$ 2,05 (m,2H), $\delta$ 1,65-1,35 (m,6H).
IR Spektrum (Film): 3480, 3080, 2980, 2920, 2860, 1645, 1440, 1380, 1240, 1090, 1030, 995, 910, 870, 740, 720, 700 $cm^{-1}$.

1,3(S)-Dihydroxynon-8-en(IV)

Zu einer Lösung von 0,61 g (3,91 mM) 2S,3S-2-Epoxy-1-hydroxynon-8-en(III) in 100 ml Tetrahydrofuran gibt man bei 0°C unter Argonschutzgas unter Rühren langsam 2 ml einer 3,4 molaren Lösung Natriumbis-(2-methoxy-ethoxy)-aluminiumhydrid(Red-Al®) (6,8 mM). Nach der Zugabe läßt man b ei 0°C 3 Stunden nachreagieren, anschließend wird die Reaktionslösung über Nacht bei 25°C belassen. Nach vorsichtiger Zugabe von 5 ml 1molarer Natriumhydroxidlösung läßt man noch 1 Stunde unter Rühren nachreagieren. Die organische Phase wird abgetrennt und die wässrige Phase wird mit 30 ml Ether gewaschen. Die vereinigten organischen Phasen werden mit gesättigter Natriumchloridlösung gewaschen, über Magnesiumsulfat getrocknet und anschließend wird das Lösungsmittel im Vakuum verdampft. Durch Kugelrohrdestillation
erhält man bei 130°C 0,5 mm Druck 0,54 g (3,42 mM) 1,3(S)-Dihydroxynon-8-en(IV) mit einem Schmelzpunkt von ca. 20°C. Dies entspricht einer Ausbeute von 89 %.
$C_9H_{18}O_2$ Analyse: berechnet: C:68,31% H:11,47%
gefunden: C:68,02% H:11,66%
$^1$H-NMR($CDCl_3$) $\delta$ 5,95-5,70 (m,1H), $\delta$ 5,10-4,90 (m,2H), $\delta$ 4,05-3,85 (br,s,2H) verschwindet durch Schütteln mit $D_2O$, $\delta$ 3,97-3,73 (m,3H) $\delta$ 2,15-2,00 (m,2H), $\delta$ 1,82-1,25 (m,8H).
IR Spektrum (Film): 3360, 3100, 2940, 1640, 1470, 1440, 1420, 1380, 1330, 1200, 1090, 1040, 1000, 915, 735 $cm^{-1}$.

1,3(S)-Dimesyloxynon-8-en(V)

Zu einer Lösung von 0,51 g (3,2 mM) 1,3(S)-Dihydroxynon-8-en(IV) in 50 ml trockenem Methylenchlorid unter Argonschutzgas werden bei -5°C 0,55 ml (7,10 mM) destilliertes Mesylchlorid und dann 1 ml (7,10 mM) destilliertes Triethylamin langsam zugegeben. Nach 3 Stunden wird das Lösungsmittel im Vakuum verdampft. Nach Zugabe von 50 ml Ether wird die Lösung mit 20 ml Wasser, 20 ml wässriger Salzsäure, 10 ml Wasser, 20 ml gesättigter Natriumbicarbonatlösung und 10 ml gesättigter Natriumchloridlösung gewaschen, mit Magnesiumsulfat getrocknet, abfiltriert, und anschließend wird das Lösungsmittel im Vakuum verdampft. Man erhält 0,97 g (3,1 mM) 1,3(S)-Dimesyloxynon-8-en(V) in einer Ausbeute von 96%. Das Produkt kann ohne weitere Reinigung weiter verwendet werden.

$C_{11}H_{22}O_6S_2$ Analyse: berechnet: C:42,02% H:7,05%
gefunden: C:41,77% H:7,07%
$^1$H-NMR ($CDCl_3$) δ 5,90-5,70 (m,1H) δ 5,10-4,95 (m,2H), δ 4,95-4,80 (quintett, J = 5 Hz, 1H) δ 3,35 (t, J = 5 Hz, 2H) δ 3,05 (s,6H) δ 2,25-1,95 (m,4H) δ 1,85-1,65 (m,2H) δ 1,55-1,32 (m,4H)
IR Spektrum (Film): 3080, 3030, 2980, 2940, 2860, 1640, 1465, 1440, 1415, 1355, 1175, 975, 915, 825, 785, 750, 742, 735, 720, 710, 703 $cm^{-1}$.

6(S),8-Dimesyloxyoctansäure(VI)

In eine Reaktionslösung aus 15 ml Tetrachlorkohlenstoff, 23 ml Wasser und 15 ml Acetonitril werden 2,10 g (6,7 mM) 1,3(S)-Dimesyloxynon-8-en vorgelegt und unter starken Rühren gibt man 2 Kristalle Rutheniumtrichloridhydrat und 5,87 g (27,4 mM) Natriumperjodat hinzu. Die Reaktionslösung läßt man noch 3 1/2 Stunden bei 25°C nachrühren. Man gibt 100 ml Methylenchlorid hinzu, extrahiert die organische Phase, trocknet über Magnesiumsulfat, filtriert und verdampft das Lösungsmittel in Vakuum. Man fügt 100 ml Ether zu dem Rückstand hinzu und filtriert die Lösung durch eine Fritte. Nach dem Verdampfen des Lösungsmittels im Vakuum erhält man nach Umkristallisieren aus Ether 1,73 g (5,21 mM) 6(S),8-Dimesyloxyoctansäure(VI) als weiße Substanz mit einem Schmelzpunkt von 54-55°C.
Die Ausbeute beträgt 78%.
$C_{10}H_{20}O_8S_2$ Analyse:berechnet: C:36,13% H:6,06%
gefunden: C:36,21% H:6,00%
$^1$H-NMR($CDCl_3$) δ 10,50-9,95 (br.,s,1H) verschwindet durch Schütteln mit $D_2O$, δ 4,94-4,77 (m,1H), δ 4,33 (t,J=5Hz,2H) δ 3,05 (s,6H) δ 2,41 (t,J=6Hz,2H) δ 2,44-2,30 (m,2H), δ 1,83-1,55 (m,4H) δ 1,55-1,30 (m,2H)
IR Spektrum (Film): 3700-2300, 3030, 2940, 2870, 1730, 1705, 1460, 1410, 1340, 1170, 1090, 970, 915, 825, 785, 735 $cm^{-1}$.

R-(+)-α-Liponsäure (VII)

In einer alkalischen Lösung aus 0,23 g (4,16 mM) Kaliumhydroxid in 5 ml Wasser löst man 1,38 g (4,16 mM) 6(S),8-Dimesyloxyoctansäure(VI). Bei schwacher Erwärmung (40°C) verdampft man im Vakuum das Wasser. Zu dem Rückstand gibt man 0,146 g (4,57 mM) Schwefelblüte und 1,10 g (4,57 mM) Natriumsulfidnonahydrat. Anschließend gibt man 10 ml Dimethylformamid zu der Reaktionsmischung und die gebildete Suspension wird 24 Stunden bei 80°C unter starkem Rühren erhitzt. Die Reaktionslösung wird in 20 g Eis/Wasser gegeben, mit ca. 5ml 3molarer Salzsäure angesäuert und
viermal mit 50 ml Chloroform extrahiert. Das Lösungsmittel wird in Vakuum verdampft, und der Rückstand in 50 ml Ether aufgenommen. Die organische Phase wird viermal mit je 10 ml Wasser gewaschen, abgetrennt, über Magnesiumsulfat getrocknet und anschließend das Lösungsmittel verdampft. Der Rückstand wird zweimal mit je 10 ml heißem Petrolether extrahiert und anschließend das Lösungsmittel im Vakuum verdampft. Nach dem Umkristallisieren aus Cyclohexan erhält man 0,44 g (2,16 mM) R(+)-α-Liponsäure in einer Ausbeute von 52 % in gelben Blättchen mit einem Schmelzpunkt von 44 - 46°C. Die optische Aktivität ergibt einen Drehwert $[\alpha]_D^{28}$ = +107° (c= 0,87 in Benzol) (Literaturwert: J.D.Elliott, J.Steele, W.S.Johnson, Tetrahedron Lett., 2535, 26, (1985) Schmelzpunkt: 43 - 45°C $[\alpha]_D^{23}$ = 102° (c = 0,91 in Benzol)

**Patentansprüche**

1. Verfahren zur Herstellung von R-(+)-α-Liponsäure und S-(-)-α-Liponsäure (Thioctsäure) entsprechend der Formel VII

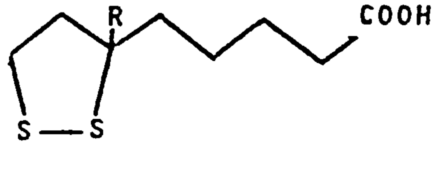

VII

wobei

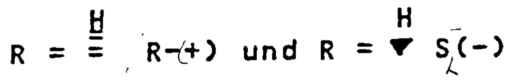

dadurch gekennzeichnet, daß man

a) eine Verbindung der Formel II

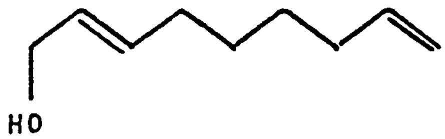

II

durch enantioselektive Epoxydation zu dem Epoxyalkohol der Formel III umsetzt,

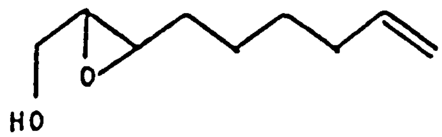

III

b) in selektiver Weise mit einem metallorganischen Reduktionsmittel zu 1,3-Dihydroxynon-8-en der Formel IV reduziert

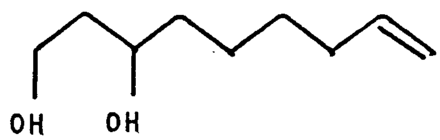

IV

c) mit einem substituierten Sulfonsäurechlorid 1,3-Disulfoxynon-8-en der Formel V herstellt

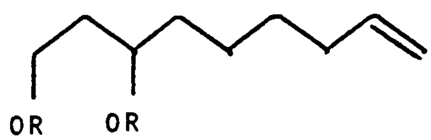

V

R=$SO_2-CH_3$,

$SO_2-C_6H_4-CH_3$

d) durch Oxidation mit Rutheniumtetroxid 6,8-Disulfoxyoctansäure der Formel VI herstellt

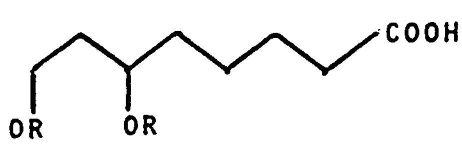

VI

R=$SO_2-CH_3$,

$SO_2-C_6H_4-CH_3$

e) und durch Reaktion mit Natriumsulfid/Schwefel in die Verbindung der Formel VII überführt.

**2.** Verfahren nach Anspruch 1
dadurch gekennzeichnet, daß man

a) eine Verbindung der Formel II

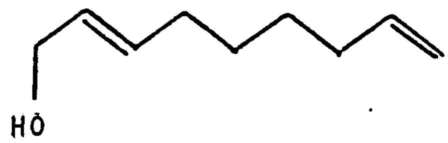

II

durch enantioselektive Epoxydation mit tertiär-Butylhydroperoxid, Tetraisopropylorthotitanat und einem chiralen Tartrat in einem Halogenkohlenwasserstoff als Lösungsmittel bei Temperaturen zwischen -25°C und 0°C zu dem Epoxyalkohol der Formel III umsetzt,

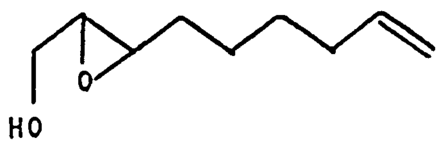

III

b) in selektiver Weise mit Natrium-bis(2-methoxyethoxy)-aluminiumhydrid in einem inerten organischen Lösungsmittel bei Temperaturen von-20°C bis +5°C zu 1,3-Dihydroxynon-8-en der Formel IV reduziert

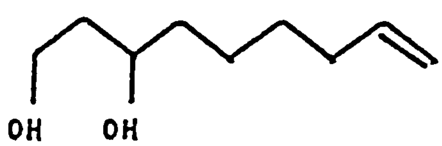

IV

c) mit Mesyl- oder Tosylchlorid in einem inerten organischen Lösungsmittel bei Temperaturen zwischen -20°C und +5°C 1,3-Disulfoxynon-8-en der Formel V herstellt

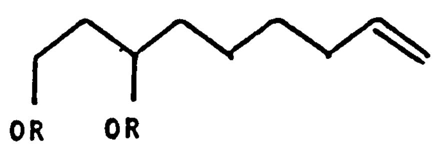

V

$R = SO_2-CH_3$,
$SO_2-C_6H_4-CH_3$

d) durch Oxidation mit in situ erzeugten katalytischen Mengen Rutheniumtetroxid in organischen Lösungsmitteln bei Temperaturen zwischen 0°C und 40°C 6,8-Disulfoxyoctansäure der Formel VI herstellt

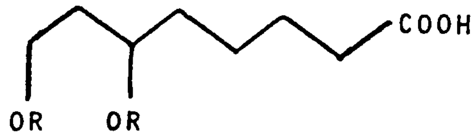

VI

R= $SO_2-CH_3$,
$SO_2-C_6H_4-CH_3$

e) und durch Reaktion mit Natriumsulfid/Schwefel in einem organischen Lösungsmittel bei Temperaturen zwischen 20° C und 100° C in die Verbindung der Formel VII überführt.

**3.** 2-Epoxy-1-hydroxynon-8-en der Formel III

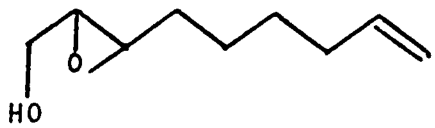

III

**4.** 1,3-Dihydroxynon-8-en der Formel IV

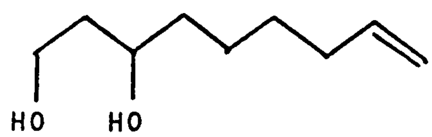

IV

**5.** 1,3-Dimesyloxynon-8-en der Formel V

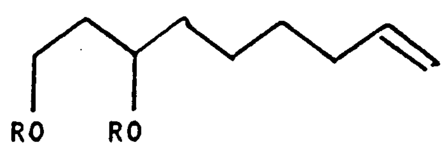

R=$SO_2CH_3$

V

**6.** 6,8-Dimesyloxyoctansäure der Formel VI

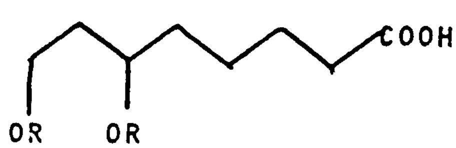

VI

R=$SO_2CH_3$

**7.** Verwendung von 2-Epoxy-1-hydroxynon-8-en der Formel III als Zwischenprodukt zur Herstellung von enantiomerenreiner R-(+)-/S-(-)-α-Liponsäure (Thioctsäure).

**8.** Verwendung von 1,3-Dihydroxynon-8-en der Formel IV als Zwischenprodukt zur Herstellung von enantiomerenreiner R-(+)-/S-(-)-α-Liponsäure (Thioctsäure).

**9.** Verwendung von 1,3-Dimesyloxynon-8-en der Formel V als Zwischenprodukt zur Herstellung von enantiomerenreiner R-(+)-/S-(-)-α-Liponsäure (Thioctsäure)

**10.** Verwendung von 6,8-Dimesyloxyoctansäure der Formel VI als Zwischenprodukt zur Herstellung von enantiomerenreiner R-(+)-/S-(-)-α-Lipsonsäure (Thioctsäure).

## Claims

**1.** A process for the production of R-(+)-α-liponic acid and S-(-)-α-liponic acid (thioctic acid) corresponding to formula VII

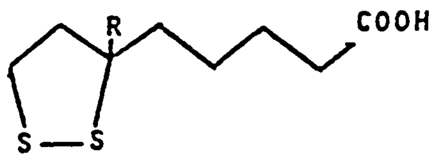

VII

in which

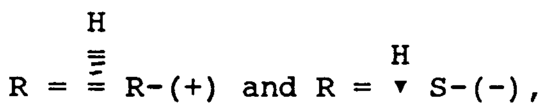

characterized in that

a) a compound corresponding to formula II

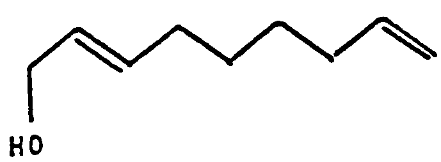

II

is reacted by enantioselective epoxidation to the epoxy alcohol corresponding to formula III

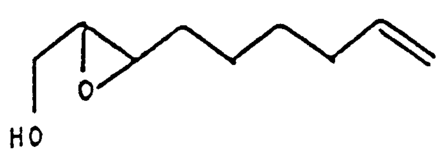

III

b) the epoxy alcohol III is selectively reduced with an organometallic reducing agent to form 1,3-dihydroxynon-8-ene corresponding to formula IV

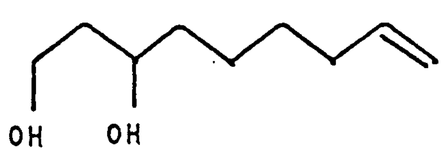

IV

c) 1,3-disulfoxynon-8-ene corresponding to formula V is prepared by reaction of IV with a substituted

sulfonic acid chloride

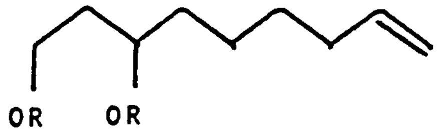

V

$R=SO_2-CH_3$,

$SO_2-C_6H_4-CH_3$

d) 6,8-disulfoxyoctanoic acid corresponding to formula IV is prepared by oxidation of V with ruthenium tetroxide

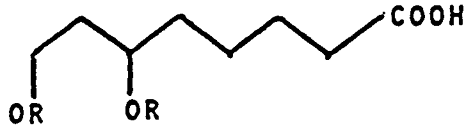

VI

$R=SO_2-CH_3$,

$SO_2-C_6H_4-CH_3$

e) and is converted into the compound corresponding to formula VII by reaction with sodium sulfide/sulfur.

**2.** A process as claimed in claim 1, characterized in that

a) a compound corresponding to formula II

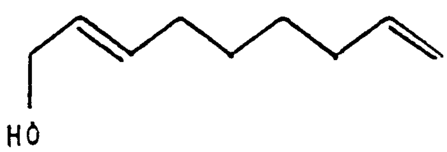

II

is reacted by enantioselective epoxidation with tert. butyl hydroperoxide, tetraisopropyl orthotitanate and a chiral tartrate in a halogenated hydrocarbon as solvent at temperatures of -25°C to 0°C to form the epoxy alcohol corresponding to formula III

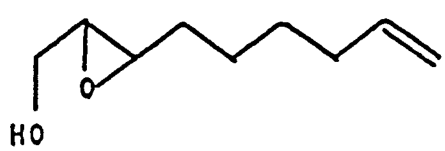

III

b) the epoxy alcohol III is selectively reduced with sodium bis-(2-methoxyethoxy)-aluminium hydride in an inert organic solvent at temperatures of -20°C to +5°C to form 1,3-dihydroxynon-8-ene corresponding to formula IV

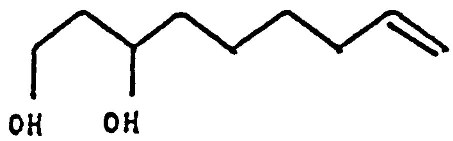

IV

c) 1,3-disulfoxynon-8-ene corresponding to formula V

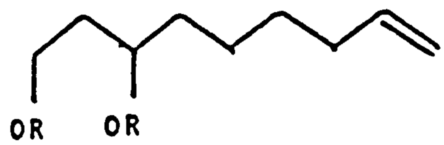

V

R= $SO_2-CH_3$, $SO_2-C_6H_4-CH_3$

is prepared by reaction of IV with mesyl or tosyl chloride in an inert organic solvent at temperatures of -20 °C to +5 °C

d) 6,8-disulfoxyoctanoic acid corresponding to formula VI

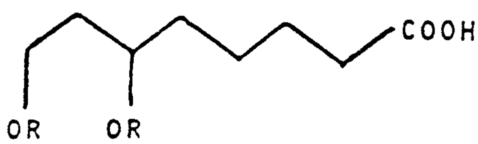

VI

R= $SO_2-CH_3$, $SO_2-C_6H_4-CH_3$

is prepared by oxidation of V with catalytic quantities of ruthenium tetroxide formed *in situ* in organic solvents at temperatures of 0 to 40 °C and

e) is converted into the compound of formula VII by reaction with sodium sulfide/sulfur in an organic solvent at temperatures of 20 to 100 °C.

**3.** 2-Epoxy-1-hydroxynon-8-ene corresponding to formula III

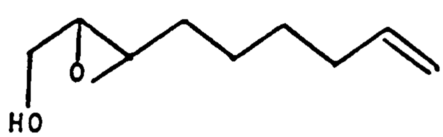

III

**4.** 1,3-Dihydroxynon-8-ene corresponding to formula IV

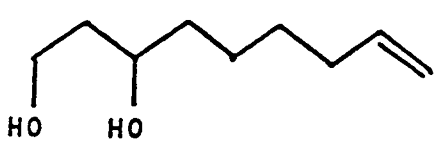

IV

**5.** 1,3-Dimesyloxynon-8-ene corresponding to formula V

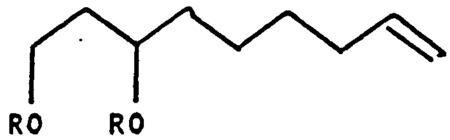

$R=SO_2CH_3$ V

6. 6,8-Dimesyloxyoctanoic acid corresponding to formula VI

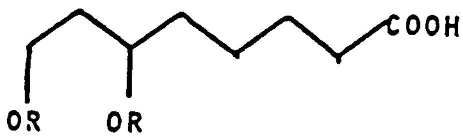

$R=SO_2CH_3$ VI

7. The use of 2-epoxy-1-hydroxynon-8-ene corresponding to formula III as an intermediate in the production of enantiomerically pure R-(+)-/S-(-)-α-liponic acid (thioctic acid).

8. The use of 1,3-dihydroxynon-8-ene corresponding to formula IV as an intermediate in the production of enantiomerically pure R-(+)-/S-(-)-α-liponic acid (thioctic acid).

9. The use of 1,3-dimesyloxynon-8-ene corresponding to formula V as an intermediate in the production of enantiomerically pure R-(+)-/S-(-)-α-liponic acid (thioctic acid).

10. The use of 6,8-dimesyloxyoctanoic acid corresponding to formula VI as an intermediate in the production of enantiomerically pure R-(+)-/S-(-)-α-liponic acid (thioctic acid)

**Revendications**

1. Procédé de préparation d'acide R-(+)-α-lipoïque et d'acide S-(-)-α-lipoïque (acide thioctique) correspondant a la formule VII

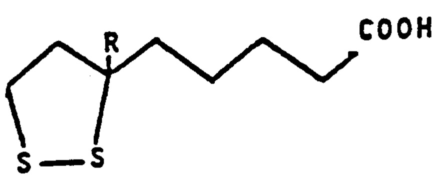

VII

dans laquelle

R = 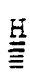 R-(+) et R = 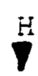 S-(-)

caractérisé en ce que

a) on transforme un composé de formule II

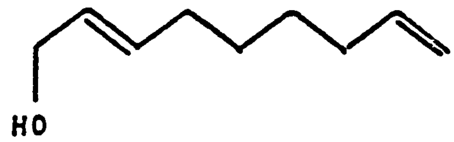

II

par époxydation énantio-sélective, en l'époxyalcool de formule III
b) on le réduit de façon sélective, avec un agent de réduction organométallique, en 1,3-dihydroxynonène-8 de formule IV

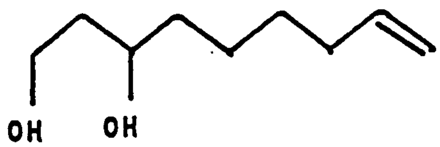

IV

c) on prépare, avec un chlorure d'acide sulfonique substitué, un 1,3-disulfoxynonène-8 de formule V

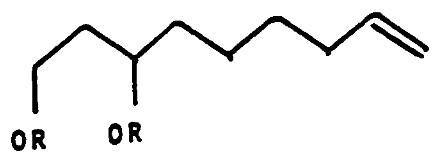

V
$R=SO_2-CH_3$,
$SO_2-C_6H_4-CH_3$

d) on prépare, par oxydation avec du tétroxyde de ruthénium, un acide 6,8-disulfoxyoctanoïque de formule VI

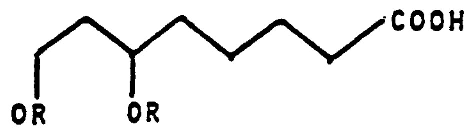

VI
$R=SO_2-CH_3$,
$SO_2-C_6H_4-CH_3$

e) et on le convertit, par réaction avec du sulfure de sodium/soufre, en le composé de formule VII.

**2.** Procédé selon la revendication 1, caractérisé en ce que
a) on transforme un composé de formule II

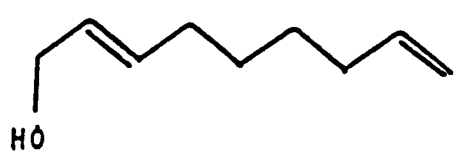

II

en l'époxyalcool de formule III par époxydation énantio-sélective avec de l'hydroperoxyde de tert.-butyle, de l'orthotitanate de tétraisopropyle et un tartrate chiral, dans un hydrocarbure halogéné servant de solvant, à une température comprise entre -25° C et 0° C

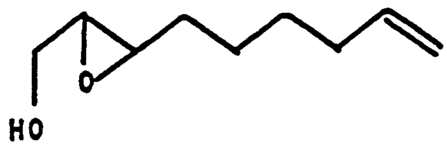

III

b) on le réduit de façon sélective en 1,3-dihydroxynonène-8 de formule IV avec de l'hydrure de bis-(2-méthoxyéthoxy)-aluminium et de sodium dans un solvant organique inerte, à une température comprise entre -20° C et +5° C

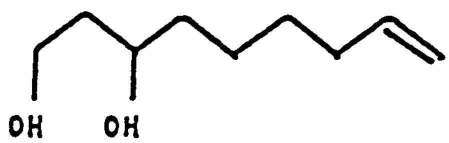

IV

c) on prépare un 1,3-disulfoxynonène-8 de formule V avec du chlorure de mésyle ou de tosyle dans un solvant organique inerte, à une température comprise entre -20° C et +5° C

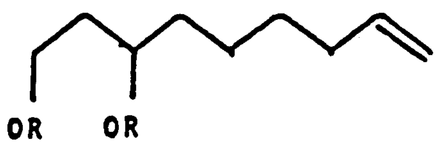

V

R= $SO_2-CH_3$, $SO_2-C_6H_4-CH_3$

d) on prépare un acide 6,8-disulfoxyoctanoïque de formule VI par oxydation avec des quantités catalytiques produites in situ de tétroxyde de ruthénium, dans des solvants organiques et à une température comprise entre 0° C et 40° C

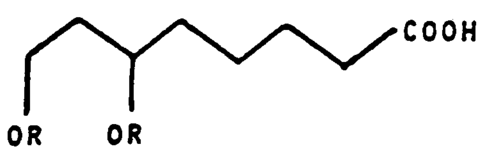

VI

R= $SO_2-CH_3$, $SO_2-C_6H_4-CH_3$

e) et on le convertit en le composé de formule VII par réaction avec du sulfure de sodium/soufre, dans un solvant organique et à une température comprise entre 20° C et 100° C.

**3.** 2-Epoxy-1-hydroxynonène-8 de formule III

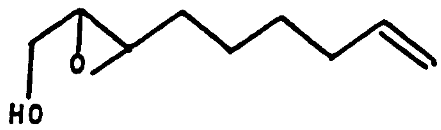

III

4. 1,3-Dihydroxynonène-8 de formule IV

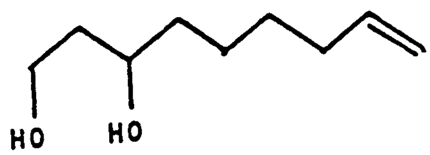

IV

5. 1,3-Dimésyloxynonène-8 de formule V

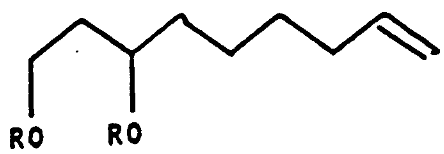

$R=SO_2CH_3$ V

6. Acide 6,8-dimésyloxyoctanoïque de formule VI

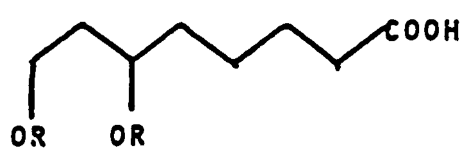

$R=SO_2CH_3$ VI

7. Utilisation du 2-époxy-1-hydroxynonène-8 de formule III somme produit intermédiaire pour la préparation des énantiomères R-(+)/S-(-) purs d'acide α-lipoïque (acide thioctique).

8. Utilisation du 1,3-dihydroxynonène-8 de formule IV comme produit intermédiaire pour la préparation des énantiomères R-(+)/S-(-) purs d'acide α-lipoïque (acide thioctique).

9. Utilisation du 1,3-dimésyloxynonène-8 de formule V comme produit intermédiaire pour la préparation des énantiomères R-(+)/S-(-) purs d'acide α-lipoïque (acide thioctique).

10. Utilisation de l'acide 6,8-dimésyloxyoctanoïque de formule VI somme produit intermédiaire pour la préparation des énantiomères R-(+)/S-(-) purs d'acide α-lipoïque (acide thioctique).